(19) **Europäisches Patentamt / European Patent Office / Office européen des brevets**

(11) **EP 3 946 491 B1**

(12) **EUROPEAN PATENT SPECIFICATION**

(45) Date of publication and mention of the grant of the patent:
**25.10.2023 Bulletin 2023/43**

(21) Application number: **20714721.6**

(22) Date of filing: **26.03.2020**

(51) International Patent Classification (IPC):
**A61L 31/10** *(2006.01)*    **A61L 31/12** *(2006.01)*
**A61L 31/18** *(2006.01)*

(52) Cooperative Patent Classification (CPC):
**A61L 31/10; A61L 31/128; A61L 31/18;**
A61L 2400/12

(86) International application number:
**PCT/NL2020/050205**

(87) International publication number:
**WO 2020/204705 (08.10.2020 Gazette 2020/41)**

(54) **USE OF ECHOGENIC COATING FOR ULTRASOUND IMAGING OF MEDICAL DEVICES IN DEEP TISSUE LAYERS**

VERWENDUNG VON ECHOGENER BESCHICHTUNG ZUR ULTRASCHALLABBILDUNG VON MEDIZINISCHEN VORRICHTUNGEN IN TIEFEN GEWEBESCHICHTEN

UTILISATION D'UN REVÊTEMENT ÉCHOGÈNE POUR L'IMAGERIE ULTRASONORE DE DISPOSITIFS MÉDICAUX DANS DES COUCHES DE TISSU PROFOND

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2019 NL 2022838**

(43) Date of publication of application:
**09.02.2022 Bulletin 2022/06**

(73) Proprietor: **Encapson B.V.**
**7521 PH Enschede (NL)**

(72) Inventors:
• **AYRES, Lee**
**6524 SE NIJMEGEN (NL)**
• **LIN, Yiyuan**
**7553 LT HENGELO (NL)**
• **BREEK, Hendricus Philip**
**1217 KZ HILVERSUM (NL)**

(74) Representative: **EP&C**
**P.O. Box 3241**
**2280 GE Rijswijk (NL)**

(56) References cited:
**WO-A1-2012/148265    WO-A1-2014/070012**
**WO-A1-2019/004828**

**Description**

**Technical Field**

The current invention relates to the use of an echogenic coating which can be applied to a medical device for improved visbility in deep tissue layers. Moreover, it relates to a method for locating the position of such a medical device at a depth of greater than 5 cm with an ultrasonic transducer.

**Background Art**

Medical ultrasonic transducers (also known as probes, both expressions are used without distinction) have been used as part of a diagnostic imaging technique that is based on the application of ultrasound. For instance, in ultrasonography transducers are used to create an image of internal body structures, e.g., to find a source of a disease. Transducers are also frequently used to examine pregnant woman. Ultrasound refers to sound waves with frequencies which are higher than those audible to humans (>20,000 Hz). Ultrasonic images, also known as sonograms, are made by sending pulses of ultrasound into tissue using an ultrasonic transducer. The ultrasound pulses echo off tissues with different reflection properties and are recorded and displayed as an image. This principle may also be used to locate or visualize an inserted device.

Coatings to enhance the echogenicity of materials are especially useful for medical devices wherein the practitioner desires to locate or visualize a device by ultrasonic imaging when the device is inserted into a body. These coatings can be applied to any device of virtually any composition.

From WO2014070012 and WO2015166081 echogenic coatings comprising solid microparticles are known. In order to obtain a sufficient contrast to noice ratio, small microparticles with specific diameter ranges were selected with a particular surface density related to the selected diameter ranges. These inventions relate to the insight that there is a discrepancy between "reflectivity" or "echogenicity" (*the amount of ultrasound signal returned to a transducer*) and "ultrasound visibility" (*the picture(s) observed on an ultrasound screen while carrying out an ultrasound-guided procedure*). More, or optimum, reflectivity is not the same as optimal ultrasound visibility. The inventors found that a good visual depiction of the contours and shape of a medical device is not simply achieved by increasing or optimizing the reflectivity. Too much signal or an overly bright picture works contra productive as it leads to over-scattering and an unclear, blurry and distorted image, particularly when the device is used in tissue.

It is stated that the coating on a medical device may comprise microparticles with a diameter between 10 and 45 $\mu$m at a density of said microparticles on the surface of the coated medical device between 45 and 450 particles/mm$^2$. In said documents it was stated that particles with larger sizes, i.e. between 45 and 53 $\mu$m were found to lead to an overestimation of the width of a marker band over the full range of the tested densities making them less desirable for clinical use. Examples were provided also with microparticles with a diameter between 38-45 $\mu$m, that were used up to a surface density of about 350 particles/mm$^2$.

Surprisingly, the coatings of WO2014070012 and WO2015166081 turned out to be less suitable for use in deeper tissue structure, i.e. structures from 5 cm and deeper into the body. The image resolution decreases as the depth increases. However, deep tissue visibility is needed, for instance - but not limited to - liver, kidney biopsies and tumour ablation therapies. Deep tissue visibility is also needed with patients that are obese, where a scan depth of greater than 5 cm, greater than 10 cm or even greater than 15 cm is needed. Thus, there is a demand for an echogenic coating that can be used for deep tissue ultrasound detection. Said echogenic coating, moreover, has to be easily applicable without adhesion of the coating to the medical device becoming a problem and or leading to discomfort when the medical device is introduced into the body.

**Summary of invention**

Surprisingly, it was found that improved visibility could be achieved when a medical device is used for deep tissue with new coating compositions as well as coating compositions that were considered less or even unsuitable for medical devices used closer to the surface. Accordingly, the use of an echogenic coating is provided, as described in claim 1, with improved ultrasound visibility when used in combination with a medical device during a clinical ultrasound procedure in deeper tissue structures, at a scan depth of greater than 5 cm, greater than 10 cm or even greater than 15 cm.

**Brief description of the drawings**

Figure 1 is an ultrasound image of a polyurethane tube coated with spherical microparticles, microspheres, together with a measurement of the mean pixel intensity of the selected region of interest (the coated PU tube).
Figure 2 is an ultrasound image of a polyurethane tube coated with spherical microparticles, microspheres, together with a measurement of the mean pixel intensity of the background.
Figure 3 are ultrasound images of coated polyurethane tubes with different coatings.

**Detailed description of the invention**

A "medical device" is defined herein as any kind of device that can be used in an animal or human body. The medical device can preferably be inserted or implanted in the body. Preferably, such medical device is an instrument used in surgery, treatment and/or diagnosis. Surgical instruments are well known in the art. Non-limiting examples of medical devices include (balloon) catheters, needles, stents, cannulas, tracheotomes, endoscopes, dilators, tubes, introducers, markers, stylets, snares, angioplasty devices, trocars, guidewires and forceps. A medical device according to the present invention is, therefore, preferably selected from the group consisting of cathethers, needles, stents, cannulas, tracheotomes, endoscopes, dilators, tubes, introducers, markers, stylets, snares, angioplasty devices, fiducials, trocars and forcepses.

Deep tissue is considered to be structures at a scan depth of at least 5 cm, preferably at least 10 cm, more preferably at least 15 cm. For instance, with obese patients it may be necessary to locate or visualize a device by ultrasonic imaging when the device is inserted at a depth of 10 to 15 cm into the body. Sonograms of deep tissue are often made with convex transducers operating at a depth range of 5-30 cm, as most linear transducers operate at depth ranges of 3-10 cm or even less.

As used herein, a coating for ultrasound detection comprises any coating that is tolerated by a human or animal body and that comprises microparticles that can be visualized, due to scattering of ultrasound waves. Typically, such coating comprises biocompatible materials that are non-toxic, hypoallergenic and stable.

An ultrasound wave (also called "an ultrasound signal" or "ultrasound") is defined as a sound pressure wave with a frequency above the audible range of normal human hearing. Typically, ultrasound waves have a frequency above 20 kHz. For imaging of medical devices, ultrasound waves with a frequency between 2 MHz and 50MHz are preferably used.

As used herein, the term "ultrasound image" means any kind of visualization of an object using ultrasound waves. Typically, reflected ultrasound waves are converted into electrical pulses which are processed and transformed into digital images. Such images are embraced by the term ultrasound image.

A "microparticle" is defined herein as a particle with a size below 250 $\mu$m, as particles greater than 250 $\mu$m are too large for practical use (the adhesion becomes problematic and the coating may appear "rough" to the patient). Moreover, the microparticle has a size greater than 10 $\mu$m. Below this value, the microparticles have insufficient visibility for practical use. Microparticles can have any shape, such as a regular shape (for instance, spherical, oval or cubical) or an irregular shape. Preferred are microspheres, which are essentially spherical in shape. The term "essentially spherical" reflects the fact that the microparticles need not be perfectly spherical as long as the distances between the centre and any point at the surface do not differ more than 50%, more preferably no more than 30%, from each other in at least 70%, preferably at least 80%, most preferably at least 90% of the particles. Gas-filled microparticles may be used, but preferably the microparticles are solid.

Echogenic microparticles are defined herein as microparticles that are able to reflect an ultrasound wave.

A monolayer, also called a single layer, is defined herein as a one-particle thick layer of particles on the surface of a device, meaning that there is on average no more than one particle on an axis perpendicular to the surface of the device. Some variations in thickness of the layer are tolerated, as long as at least 70%, preferably at least 80%, most preferably at least 90% of the coated surface of a device is coated with a single layer of particles.

Median values are defined as the value where half of the population resides above this point, and half resides below this point. The $D_{50}$ is the size in $\mu$m that splits the particle size distribution, based on volume distribution in half, with 50% of the microparticles having a particle size above and 50 % below this diameter.

A microparticle with a diameter between a given range is defined herein as a microparticle of which the diameter lies within the recited range, including the upper value of the range. For instance, a microparticle with a diameter between 38 and 45 $\mu$m may have a diameter of greater than 38 $\mu$m, a diameter of 45 $\mu$m, or a diameter with a value anywhere within this range. The diameter of a non-spherical particle is defined as the diameter of the smallest sphere that can enclose the particle in its entirety.

Contrast measurements are a quantitative method to evaluate the ultrasound visibility of the medical device. The method compares the mean pixel intensity of the medical device, against the mean pixel intensity of the surrounding background image. Contrast values of the coated area are used to quantitatively assess ultrasound visibility of different coatings. Pixel intensity is measured using imaged, a Java-based image processing program developed at the National Institutes of Health and the Laboratory for Optical and Computational Instrumentation (LOCI, University of Wisconsin), or a similar image processing program. If the image quality or "contrast" is below 25, then the device is not visible enough for clinical use.

From the recorded images, the contrast is determined by comparing the mean pixel intensity of the coated objects to the mean pixel intensity obtained for the surrounding background, according to the following equation:

$$Contrast = P_{ROI} - P_{bkg}$$

where

$$P_{ROI} = mean\ pixel\ intensity\ of\ the\ region\ of\ interest$$

$$P_{bkg} = mean\ pixel\ intensity\ of\ the\ surrounding\ background$$

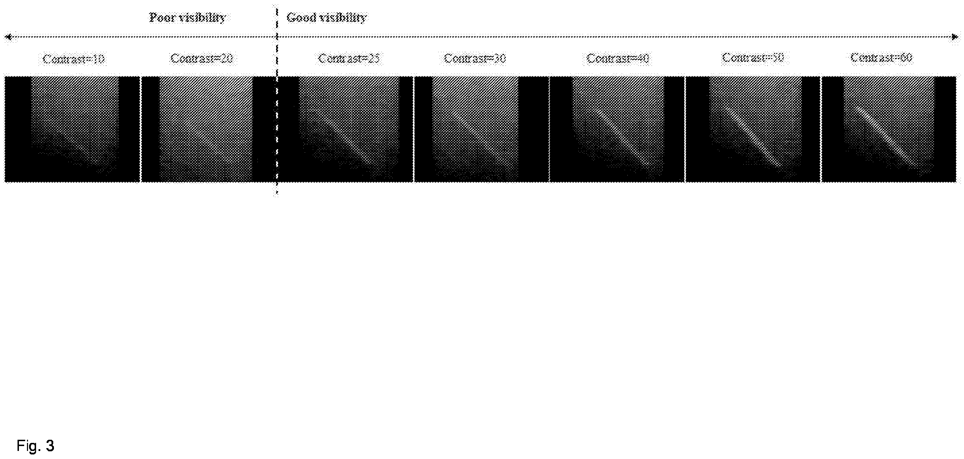

Examples of the obtained ultrasound images with contrast from 10 to 50 are shown in FIG. 3. As can be seen in FIG. 3, a minimum contrast of 25 is required in order to achieve good ultrasound visibility. Having a contrast of 30 or even 40 is preferred.

In order to locate or visualize a device by ultrasonic imaging when the device is inserted into the body, an ultrasound probe is used. In addition to linear ultrasound probes also convex (curved) type probes are known, as well as phased (sector) type probes. Convex probes find use in the diagnoses of organs, transvaginal and transrectal applications and abdominal application. A convex probe is therefore frequently used for examinations in deeper tissue structures, e.g., tissue at least 5 cm deep. The piezoelectric crystal arrangement is curvilinear and the beam is convex. The radius of curvature may vary from 5 to 80 mm. The convex probe makes use of lower frequencies, typically in the range of 2.5 - 7.5 MHz. The coating of the present invention, and medical devices comprising the new coating, can be accurately located using convex probes. The present invention therefore also includes a method for locating or visualizing a device that is inserted into deep tissue structures of a body by ultrasonic imaging with the use of an ultrasound probe, preferably a convex probe. Note that a linear ultrasound probe may be used as well.

The particle size distribution of the microparticles used in the present invention may be relatively broad, as long as the particles are at least 10 $\mu$m in diameter and at most 250 $\mu$m in diameter. The inventors found that there is a non-linear relationship between the diameter of the microparticles and the surface density (minimum and maximum). Below the lower limits of the surface density insufficient visibility is obtained which adversely affect the accuracy of localizing the coated medical device. Above the upper limits a range of problems occur, such as, over exposure causing the contours of the medical device to become blurred and less defined which will also adversely affect the accuracy of localizing the coated medical device. Above the upper limit there are problems related to adhesion of the coating to the medical device and abrasiveness of the coated medical device.

Within the limits provided above, it has been determined that for improved visibility in deep tissue structures the relationship between the particle size, expressed as $D_{50}$, and the surface density is as follows (for a contrast of at least 25):

| | Particle size ($\mu$m) | surface density (microparticles/mm$^2$) | |
|---|---|---|---|
| Condition | | Lower limit | Upper limit |
| I | $10 < D_{50} \leq 22$ | 362 | 2080 |
| II | $22 < D_{50} \leq 27$ | 478 | 1626 |
| III | $27 < D_{50} \leq 32$ | 467 | 1692 |
| IV | $32 < D_{50} \leq 38$ | 235 | 1636 |
| V | $38 < D_{50} \leq 45$ | 256 | 1459 |
| VI | $45 < D_{50} \leq 53$ | 218 | 1594 |

More preferably, the relationship between the diameter, expressed as $D_{50}$, and the surface density is as follows (for a contrast of at least 30):

|  | Particle size ($\mu$m) | surface density (microparticles/mm$^2$) | |
|---|---|---|---|
| Condition |  | Lower limit | Upper limit |
| I | $10 < D_{50} \leq 22$ | 856 | 2080 |
| II | $22 < D_{50} \leq 27$ | 1218 | 1626 |
| III | $27 < D_{50} \leq 32$ | 1074 | 1692 |
| IV | $32 < D_{50} \leq 38$ | 350 | 1636 |
| V | $38 < D_{50} \leq 45$ | 291 | 1459 |
| VI | $45 < D_{50} \leq 53$ | 318 | 1594 |

Still more preferably, the relationship between the diameter, expressed as $D_{50}$, and the surface density is as follows (for a contrast of at least 40):

|  | Particle size ($\mu$m) | surface density (microparticles/mm$^2$) | |
|---|---|---|---|
| Condition |  | Lower limit | Upper limit |
| III | $27 < D_{50} \leq 32$ | 1380 | 1692 |
| IV | $32 < D_{50} \leq 38$ | 1153 | 1636 |
| V | $38 < D_{50} \leq 45$ | 370 | 1459 |
| VI | $45 < D_{50} \leq 53$ | 370 | 1594 |

Each of the conditions concerns microparticles wherein 50% (by volume) of all microparticles have a diameter between the lower limit of 10 $\mu$m and the actual value of the $D_{50}$, whereas the remaining 50% of microparticles have a diameter between the actual value of the $D_{50}$ and the upper limit of 250 $\mu$m. The distribution may therefore be relatively broad. Microparticles with a normal diameter distribution, preferably a narrow diameter distribution, wherein at least 60% (by volume) of all microparticles have a diameter of $D_{50} \pm 5$ $\mu$m may be very suitably be used. For instance, microparticles may be used with a very narrow diameter distribution, wherein at least 70% (by volume), preferably at least 80%, more preferably at least 90%, still more preferably at least 95% of all microparticles have a diameter of $D_{50} \pm 5$ $\mu$m. Alternatively, "gap-graded" or multimodal selections of microparticles may be used, whereby "ordinary" suitable grades of different conditions are mixed, thereby creating a more complex diameter distribution. These gap-graded or multimodal grades may be used at the surface density set out above for the $D_{50}$ of these complex grades.

Surprisingly, the selection of the microparticles within the above subranges also provides for smooth coatings, whereby abrasion and adhesion problems, that are believed to be caused by the formation of bilayers of particles, is avoided. Moreover, a bilayer may appear "rough" to the patient and may impact the adhesion of the coating to the medical device. If a coating with a bilayer is bent, the coating will crack and come off, or if inserted into a body, the coating adhesion will be compromised and the coating will be abraded and flake off the surface of the device. This obviously needs to be avoided.

A medical device according to the present invention can be coated with various kinds of microparticles that are visible with ultrasound. Such microparticles are known in the art.

Typically, said microparticles comprise a material selected from the group consisting of ceramics, glasses, silicates, metals and any combination thereof. Such materials provide for optimal ultrasound response in common coating matrices.

Preferably, said microparticles are made from glass, more preferably from silica-based glass, most preferably from soda-lime glass. Such particles have a high acoustic impedance and therefore good ultrasound visibility in common coating matrices. Moreover, they are relatively easy to manufacture as compared to microparticles of other materials.

The above described microparticles need to be embedded in a coating matrix which adheres to both the medical device and the microparticles. In principle, any coating matrix capable of adhereing to both the microparticles and to a medical device can be used. The coating matrix should be suitable for in-vivo use. Such coating is preferably non-toxic, hypo-allergenic and stable. Preferably, the coating matrix comprises a polymer material.

As polymer material, various polymers and combinations of polymers may be used, which includes homopolymers, copolymers, terpolymers, and block copolymers. Preferably, the polymer material is selected from poly(ether sulfones), polyurethanes, polyacrylates, polymethacrylates, polyamides, polycarbonates, polyepoxides, polyethers, polyimides, polyesters, fluorinated polyolefins, polystyrenes and combinations thereof.

Preferably, said medical device is selected from the group consisting of cathethers, needles, stents, cannulas, tracheotomes, endoscopes, dilators, tubes, introducers, markers, stylets, snares, angioplasty devices, fiducials, trocars and forcepses. These medical devices may comprise a plastic or metallic surface.

Methods for providing echogenic coatings are well-known. A medical device may for instance be coated with the microparticles by dip coating, spray coating, pad printing, roller coating, printing, painting or inkjet printing. Reference is for instance made to US patents 5,289,831, 5,921,933, and 6,506, 156, to international patent application WO 2007/089761 and to Ultrasound in Medicine and Biology, Vol. 32, No. 8, pp. 1247-1255, 2006, which describe methods for preparing echogenic particles and coatings. Such coating is preferably biocompatible, non-toxic, hypo-allergenic and stable.

The invention thus provides use of an echogenic coating composition on a medical device to be inserted into a body at depths deeper than 5 cm, comprising the new echogenic coating compositions. It also provides a medical device for use at scan depths greater than 5 cm, preferably greater than 10 cm, more preferably greater than 15 cm, comprising the new echogenic coating composition. Moreover, the present invention provides a method for ultrasound detection of a medical device at a scan depth greater than 5 cm, preferably greater than 10 cm, more preferably greater than 15 cm, by providing the medical device with the echogenic coating composition of the present invention. Particularly attractive is the method wherein use is made of a linear or convex probe, preferably a convex probe. The convex probe preferably has a radius of curvature between 5 and 80 mm, more preferably between 20 and 65 mm. Suitably, use is made of a convex probe operating with ultrasound waves with a frequency of between 2.5 and 7.5 MHz. The invention also provides an echogenic assembly comprising a medical device for use at scan depths greater than 5 cm, preferably greater than 10 cm, more preferably greater than 15 cm, comprising the echogenic coating composition of the present invention, and a convex probe. In the echogenic assembly the coating of the medical device is specifically adapted for localization by the convex probe.

The invention may be better understood with reference to the drawings.

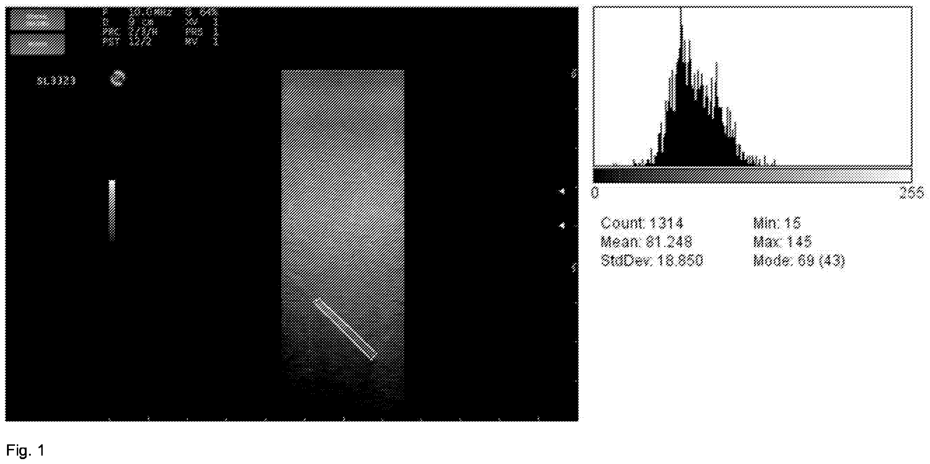

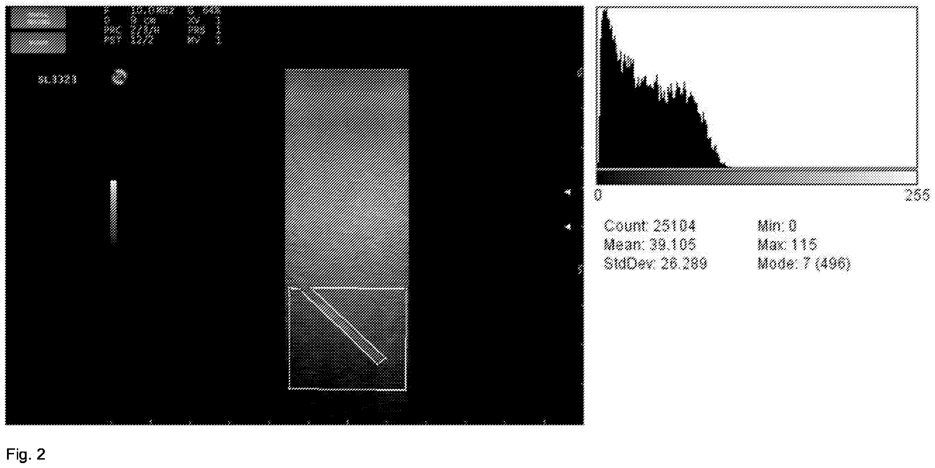

Fig. 1 and 2 show an example of the pixel intensity measurement. In Fig. 1 the pixel intensity measurement is performed of the region of interest ($P_{roi}$). In Fig. 2 the pixel intensity measurement is performed of the background ($P_{bkg}$). The visibility is determined by the contrast, based on the equation provided before, Contrast = $P_{roi}$ - $P_{bkg}$, for a coated PU tube placed at 8 cm at 45° in an echogenic gel, whereby the ultrasound images of the coated tubes were obtained using an Esaote Mylab One touch with a linear probe. Scanning frequency was 10 MHz and the brightness gain 64%.

Fig. 3 shows ultrasound images of the coated area with measured contrast from 10 - 60. It is clear that images with higher contrast give better visibility. 25 is the minimum contrast needed for the clinician to be able to accurately localize the coated device.

EXPERIMENTAL

Contrast measurements have been performed on polyurethane tubes on which a thin film of coating containing microparticles has been applied using a dip coater. The surface density of microparticles on the PU tubes has been determined by counting the number of microparticles per $mm^2$ under a microscope. The contrast measurements have been performed in an ultrasound phantom as a test medium, using a linear array ultrasound probe operating at 10 MHz. The tubes were inserted at an approximate angle of 45°, relative to the ultrasound probe. The distal end of the polyurethane tube was positioned at a depth of 8 cm inside the test medium.

A series of coated PU tubes were prepared with an increasing number of particles on the surface for each of the 6 particle size conditions. The ultrasound contrast of each coated PU tube was measured according to the method described above. The data are indicated in Table 1. In the table the surface density of microparticles per square millimetre is provided for a contrast of at least 25, at least 30 or even at least 40. Moreover, an upper limit is provided where monolayer is still visible, and an upper limit beyond which bilayer formation will occur. Since problems of adhesion and abrasiveness are to be avoided upper limits that are close to the upper limit known to have a monolayer are preferred.

Table 1

| Condition | Particle size ($\mu$m) | Contrast >25 | Contrast >30 | Contrast >40 | Maximum monolayer | Bilayer |
|---|---|---|---|---|---|---|
| I | 10 < $D_{50}$ ≤22 | 362 | 856 | n.a. | 1980 | 2180 |
| II | 22 < $D_{50}$ ≤27 | 478 | 1218 | n.a. | 1541 | 1712 |
| III | 27 < $D_{50}$ ≤32 | 467 | 1074 | 1380 | 1552 | 1833 |

(continued)

| Condition | Particle size ($\mu$m) | Contrast >25 | Contrast >30 | Contrast >40 | Maximum monolayer | Bilayer |
|---|---|---|---|---|---|---|
| IV | $32 < D_{50} \leq 38$ | 235 | 350 | 1153 | 1511 | 1762 |
| V | $38 < D_{50} \leq 45$ | 256 | 291 | 370 | 1373 | 1546 |
| VI | $45 < D_{50} \leq 53$ | 218 | 318 | 370 | 1478 | 1711 |

**Claims**

1. Use of an echogenic coating composition on a medical device to be inserted into a body at depths greater than 5 cm, comprising:

   (i) a polymer matrix and
   (ii) an amount of ultrasound-reflective microparticles having a diameter that is at least 10 and at most 250 $\mu$m in size, wherein the relationship between the particle size, expressed as $D_{50}$, and the surface density is as follows:

|  | Particle size ($\mu$m) | surface density (microparticles/mm$^2$) | |
|---|---|---|---|
| Condition |  | Lower limit | Upper limit |
| I | $10 < D_{50} \leq 22$ | 362 | 2080 |
| II | $22 < D_{50} \leq 27$ | 478 | 1626 |
| III | $27 < D_{50} \leq 32$ | 467 | 1692 |
| IV | $32 < D_{50} \leq 38$ | 235 | 1636 |
| V | $38 < D_{50} \leq 45$ | 256 | 1459 |
| VI | $45 < D_{50} \leq 53$ | 218 | 1594 |

   wherein the use is non-surgical.

2. The use of an echogenic coating composition as claimed in claim 1, wherein the relationship between the particle size, expressed as $D_{50}$, and the surface density is as follows:

|  | Particle size ($\mu$m) | surface density (microparticles/mm$^2$) | |
|---|---|---|---|
| Condition |  | Lower limit | Upper limit |
| I | $10 < D_{50} \leq 22$ | 856 | 2080 |
| II | $22 < D_{50} \leq 27$ | 1218 | 1626 |
| III | $27 < D_{50} \leq 32$ | 1074 | 1692 |
| IV | $32 < D_{50} \leq 38$ | 350 | 1636 |
| V | $38 < D_{50} \leq 45$ | 291 | 1459 |
| VI | $45 < D_{50} \leq 53$ | 318 | 1594 |

3. The use of an echogenic coating composition as claimed in claim 1, wherein the relationship between the particle size, expressed as $D_{50}$, and the surface density is as follows:

| | Particle size ($\mu$m) | surface density (microparticles/mm$^2$) | |
|---|---|---|---|
| Condition | | Lower limit | Upper limit |
| III | $27 < D_{50} \leq 32$ | 1380 | 1692 |
| IV | $32 < D_{50} \leq 38$ | 1153 | 1636 |
| V | $38 < D_{50} \leq 45$ | 370 | 1459 |
| VI | $45 < D_{50} \leq 53$ | 370 | 1594 |

4. The use of an echogenic coating composition of any one of claims 1-3, wherein the polymer material is selected from poly(ether sulfones), polyurethanes, polyacrylates, polymethacrylates, polyamides, polycarbonates, polyepoxides, polyethers, polyimides, polyesters, fluorinated polyolefins, polystyrenes and combinations thereof.

5. The use of an echogenic coating composition of any one of claims 1-4, wherein the microparticles are spherical.

6. The use of an echogenic coating composition of any one of claims 1-5, wherein the microparticles are made of glass.

7. A method for ultrasound detection of a medical device at a scan depth greater than 5 cm, preferably greater than 10 cm, more preferably greater than 15 cm, by providing the medical device with the echogenic coating composition defined in any one of claims 1-6, wherein the method is non-surgical.

8. The method of claim 7, wherein use is made of a linear or convex probe, preferably a convex probe.

9. The method of claim 8, wherein use is made of a convex probe with a radius of curvature between 5 and 80 mm.

10. The method of claim 8 or 9, wherein use is made of a convex probe operating with ultrasound waves with a frequency of between 2.5 and 7.5 MHz.

11. An echogenic assembly comprising:
a medical device for use at scan depths greater than 5 cm, preferably greater than 10 cm, more preferably greater than 15 cm, comprising the echogenic coating composition defined in any one of claims 1-6, and a convex probe.


**Patentansprüche**

1. Verwendung einer echogenen Beschichtungszusammensetzung auf einem medizinischen Gerät, das in Tiefen von mehr als 5 cm in einen Körper eingeführt werden soll, umfassend:

(i) eine Polymermatrix und
(ii) eine Menge ultraschallreflektierender Mikropartikel mit einem Durchmesser, der mindestens 10 und höchstens 250 $\mu$m groß ist, wobei der Zusammenhang zwischen der Partikelgröße, ausgedrückt als $D_{50}$, und der Oberflächendichte wie folgt ist:

| | Partikelgröße ($\mu$m) | Oberflächendichte (Mikropartikel/mm$^2$) | |
|---|---|---|---|
| Bedingung | | Untergrenze | Obergrenze |
| I | $10 < D_{50} \leq 22$ | 362 | 2080 |
| II | $22 < D_{50} \leq 27$ | 478 | 1626 |
| III | $27 < D_{50} \leq 32$ | 467 | 1692 |
| IV | $32 < D_{50} \leq 38$ | 235 | 1636 |

(fortgesetzt)

| Bedingung | Partikelgröße ($\mu$m) | Oberflächendichte (Mikropartikel/mm$^2$) | |
|---|---|---|---|
| | | Untergrenze | Obergrenze |
| V | $38 < D_{50} \leq 45$ | 256 | 1459 |
| VI | $45 < D_{50} \leq 53$ | 218 | 1594 |

wobei die Verwendung nicht-chirurgisch ist.

2. Verwendung einer echogenen Beschichtungszusammensetzung wie in Anspruch 1 beansprucht, wobei die Beziehung zwischen der Partikelgröße, ausgedrückt als $D_{50}$, und der Oberflächendichte wie folgt ist:

| Bedingung | Partikelgröße ($\mu$m) | Oberflächendichte (Mikropartikel/mm$^2$) | |
|---|---|---|---|
| | | Untergrenze | Obergrenze |
| I | $10 < D_{50} \leq 22$ | 856 | 2080 |
| II | $22 < D_{50} \leq 27$ | 1218 | 1626 |
| III | $27 < D_{50} \leq 32$ | 1074 | 1692 |
| IV | $32 < D_{50} \leq 38$ | 350 | 1636 |
| V | $38 < D_{50} \leq 45$ | 291 | 1459 |
| VI | $45 < D_{50} \leq 53$ | 318 | 1594 |

3. Verwendung einer echogenen Beschichtungszusammensetzung wie in Anspruch 1 beansprucht, wobei die Beziehung zwischen der Partikelgröße, ausgedrückt als $D_{50}$, und der Oberflächendichte wie folgt ist:

| Bedingung | Partikelgröße ($\mu$m) | Oberflächendichte (Mikropartikel/mm$^2$) | |
|---|---|---|---|
| | | Untergrenze | Obergrenze |
| III | $27 < D_{50} \leq 32$ | 1380 | 1692 |
| IV | $32 < D_{50} \leq 38$ | 1153 | 1636 |
| V | $38 < D_{50} \leq 45$ | 370 | 1459 |
| VI | $45 < D_{50} \leq 53$ | 370 | 1594 |

4. Verwendung einer echogenen Beschichtungszusammensetzung nach einem der Ansprüche 1-3, wobei das Polymermaterial aus Poly(ethersulfonen), Polyurethanen, Polyacrylaten, Polymethacrylaten, Polyamiden, Polycarbonaten, Polyepoxiden, Polyethern, Polyimiden, Polyestern, fluorierten Polyolefinen, Polystyrolen und Kombinationen davon ausgewählt ist.

5. Verwendung einer echogenen Beschichtungszusammensetzung nach einem der Ansprüche 1-4, wobei die Mikropartikel kugelförmig sind.

6. Verwendung einer echogenen Beschichtungszusammensetzung nach einem der Ansprüche 1-5, wobei die Mikropartikel aus Glas sind.

7. Verfahren zur Ultraschallerkennung eines medizinischen Geräts bei einer Scantiefe von mehr als 5 cm, bevorzugt mehr als 10 cm, stärker bevorzugt mehr als 15 cm, durch Versehen des medizinischen Geräts mit der in einem der Ansprüche 1-6 definierten echogenen Beschichtungszusammensetzung, wobei das Verfahren nicht-chirurgisch ist.

8. Verfahren nach Anspruch 7, wobei eine lineare oder konvexe Sonde, vorzugsweise eine konvexe Sonde, verwendet wird.

**9.** Verfahren nach Anspruch 8, wobei eine konvexe Sonde mit einem Krümmungsradius zwischen 5 und 80 mm verwendet wird.

**10.** Verfahren nach Anspruch 8 oder 9, wobei eine konvexe Sonde verwendet wird, die mit Ultraschallwellen mit einer Frequenz zwischen 2,5 und 7,5 MHz arbeitet.

**11.** Echogene Baugruppe umfassend:
ein medizinisches Gerät zur Verwendung bei Scantiefen von mehr als 5 cm, bevorzugt mehr als 10 cm, stärker bevorzugt mehr als 15 cm, umfassend die in einem der Ansprüche 1-6 definierte echogene Beschichtungszusammensetzung und eine konvexe Sonde.

**Revendications**

**1.** Utilisation d'une composition de revêtement échogène sur un dispositif médical destiné à être inséré dans un corps à des profondeurs supérieures à 5 cm, comprenant :

(i) une matrice polymère et
(ii) une quantité de microparticules réfléchissant les ultrasons dont le diamètre est compris entre au moins 10 et au plus 250 $\mu$m, dans laquelle la relation entre la taille des particules, exprimée en $D_{50}$ et la densité de surface étant la suivante

|  | Taille des particules ($\mu$m) | Densité de surface (microparticules/mm$^2$) | |
|---|---|---|---|
| Condition | | Limite inférieure | Limite supérieure |
| I | $10 < D_{50} \leq 22$ | 362 | 2080 |
| II | $22 < D_{50} \leq 27$ | 478 | 1626 |
| III | $27 < D_{50} \leq 32$ | 467 | 1692 |
| IV | $32 < D_{50} \leq 38$ | 235 | 1636 |
| V | $38 < D_{50} \leq 45$ | 256 | 1459 |
| VI | $45 < D_{50} \leq 53$ | 218 | 1594 |

dans lequel l'utilisation est non chirurgicale.

**2.** Utilisation d'une composition de revêtement échogène selon la revendication 1, dans laquelle la relation entre la taille des particules, exprimée en $D_{50}$, et la densité de surface est la suivante :

|  | Taille des particules ($\mu$m) | Densité de surface (microparticules/mm$^2$) | |
|---|---|---|---|
| Condition | | Limite inférieure | Limite supérieure |
| I | $10 < D_{50} \leq 22$ | 856 | 2080 |
| II | $22 < D_{50} \leq 27$ | 1218 | 1626 |
| III | $27 < D_{50} \leq 32$ | 1074 | 1692 |
| IV | $32 < D_{50} \leq 38$ | 350 | 1636 |
| V | $38 < D_{50} \leq 45$ | 291 | 1459 |
| VI | $45 < D_{50} \leq 53$ | 318 | 1594 |

**3.** Utilisation d'une composition de revêtement échogène selon la revendication 1, dans laquelle la relation entre la taille des particules, exprimée en $D_{50}$, et la densité de surface est la suivante :

| | Taille des particules ($\mu$m) | Densité de surface (microparticules/mm$^2$) | |
|---|---|---|---|
| Condition | | Limite inférieure | Limite supérieure |
| III | $27 < D_{50} \leq 32$ | 1380 | 1692 |
| IV | $32 < D_{50} \leq 38$ | 1153 | 1636 |
| V | $38 < D_{50} \leq 45$ | 370 | 1459 |
| VI | $45 < D_{50} \leq 53$ | 370 | 1594 |

4. Utilisation d'une composition de revêtement échogène selon l'une quelconque des revendications 1 à 3, dans laquelle le matériau polymère est choisi parmi les poly(éther sulfones), les polyuréthanes, les polyacrylates, les polyméthacrylates, les polyamides, les polycarbonates, les polyépoxydes, les polyéthers, les polyimides, les polyesters, les polyoléfines fluorées, les polystyrènes et des combinaisons de ceux-ci.

5. Utilisation d'une composition de revêtement échogène selon l'une des revendications 1 à 4, dans laquelle les microparticules sont sphériques.

6. Utilisation d'une composition de revêtement échogène selon l'une des revendications 1 à 5, dans laquelle les microparticules sont en verre.

7. Méthode de détection par ultrasons d'un dispositif médical à une profondeur de balayage supérieure à 5 cm, de préférence supérieure à 10 cm, de préférence supérieure à 15 cm, en dotant le dispositif médical de la composition de revêtement échogène définie selon l'une quelconque des revendications 1 à 6, dans laquelle la méthode n'est pas chirurgicale.

8. Méthode de la revendication 7, dans laquelle on utilise une sonde linéaire ou convexe, de préférence une sonde convexe.

9. Méthode de la revendication 8, dans laquelle on utilise une sonde convexe dont le rayon de courbure est compris entre 5 et 80 mm.

10. Méthode de la revendication 8 ou 9, dans laquelle on utilise une sonde convexe fonctionnant avec des ondes ultrasonores d'une fréquence comprise entre 2,5 et 7,5 MHz.

11. Ensemble échogène comprenant :
un dispositif médical destiné à être utilisé à des profondeurs de balayage supérieures à 5 cm, de préférence supérieures à 10cm, de préférence supérieure à 15 cm, comprenant la composition de revêtement échogène définie selon l'une quelconque des revendications 1 à 6, et une sonde convexe.

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014070012 A
- WO 2015166081 A
- US 5289831 A
- US 5921933 A
- US 6506156 B
- WO 2007089761 A

**Non-patent literature cited in the description**

- *Ultrasound in Medicine and Biology,* 2006, vol. 32 (8), 1247-1255